(19) Europäisches Patentamt
European Patent Office
Office européen des brevets

(11) Numéro de publication: **0 359 647**
**A1**

(12) # DEMANDE DE BREVET EUROPEEN

(21) Numéro de dépôt: **89402472.8**

(22) Date de dépôt: **11.09.89**

(51) Int. Cl.5: **C 07 D 489/08**

(30) Priorité: **13.09.88 FR 8811930**

(43) Date de publication de la demande:
**21.03.90 Bulletin 90/12**

(84) Etats contractants désignés:
**AT BE CH DE ES FR GB GR IT LI LU NL SE**

(71) Demandeur: **SANOFI**
**40, Avenue George V**
**F-75008 Paris (FR)**

(72) Inventeur: **Andre, Jean-Daniel**
**Impasse des Combes**
**F-04200 Sisteron (FR)**

**Dormoy, Jean-Robert**
**9 Avenue du Thor**
**F-04200 Sisteron (FR)**

**Heymes, Alain**
**5 rue Frédéric Mistral**
**F-04200 Sisteron (FR)**

(74) Mandataire: **Le Guen, Gérard et al**
**CABINET LAVOIX 2, place d'Estienne d'Orves**
**F-75441 Paris Cédex 09 (FR)**

(54) **Procédé de préparation de dérivés de morphinane.**

(57) L'invention a pour objet un procédé de préparation de dérivés de morphinane de formule générale

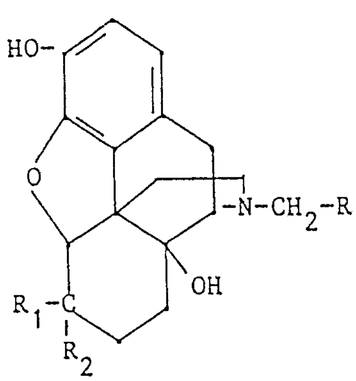

I

par déméthylation d'un composé 3-méthoxylé avec un acide sulfonique choisi parmi l'acide méthanesulfonique et l'acide trifluorométhanesulfonique en présence d'un sulfure.

EP 0 359 647 A1

**Description**

**Procédé de préparation de dérivés de morphinane**

La présente invention se rapporte, d'une manière généale, à un nouveau procédé de préparation de dérivés de morphinane.

En particulier, l'invention concerne un nouveau procédé pour la préparation de dérivés de morphinane de formule générale :

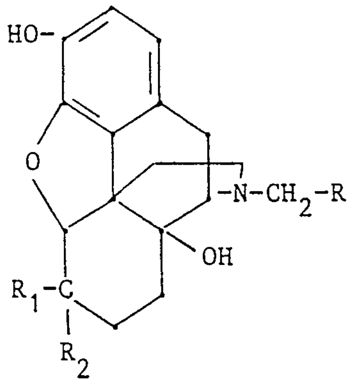

I

dans laquelle R représente l'hydrogène, un radical alkyle en $C_1$-$C_4$, cycloalkyle en $C_3$-$C_4$, alkényle en $C_2$-$C_4$ ou alkynyle en $C_2$-$C_4$, $R_1$ représente l'hydrogène, $R_2$ représente le radical hydroxyle ou $R_1$ et $R_2$, forment ensemble, avec l'atome de carbone auquel ils sont attachés, le groupe C=O, ainsi que de leurs sels pharmaceutiquement acceptables.

Ainsi, le procédé de l'invention permet notamment la préparation de la naloxone (R représente un radical vinyle, $CR_1R_2$ représente C=O), de la nalmexone (R représente un groupe méthyl-2 propèn-1 yle, $CR_1R_2$ représente C=O) ou de la naltrexone (R représente un radical cyclopropyle, $CR_1R_2$ représente C=O) ou de la nalbuphine (R représente un radical cyclobutyle, $R_1$ représente H et $R_2$ représente OH), ces produits étant largement connus pour leurs propriétés antagonistes des nacotiques, ainsi d'ailleurs que la préparation de l'oxymorphone, composé utilisé pour ses propriétés analgésiques.

Parmi les composés de formule I ceux dans lesquels R représente l'hydrogène ou un radical vinyle ou cyclopropyle et $CR_1R_2$ représente le groupement C=O constituent des composés préférés.

En outre, l'invention se rapporte préférentiellement à la préparation du composé de formule I dans laquelle R représente l'hydrogène et $CR_1R_2$ représente C=O ainsi que du composé de formule I dans laquelle R représente le radical vinyle et $CR_1R_2$ représente le groupement C=O.

Depuis une dizaine d'années, le tribomure de bore constitue le réactif de choix pour déméthyler l'éther méthylique situé en position 3 de nombreux dérivés morphiniques comportant le squelette :

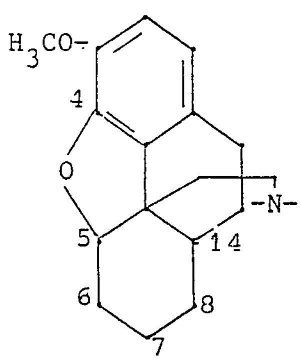

I a

en particulier les dérivés comportant un groupement hydroxyle en position 14.

Ce réactif présente en fait une grande sélectivité et peut être employé dans des conditions douces. Si l'utilisation du tribromure de bore tend à se généraliser de plus en plus pour ce genre de réaction, celui-ci n'est toutefois pas sans présenter certains problèmes au niveau industriel étant donné sa toxicité, son caractère corrosif important et sa manipulation difficile.

La recherche d'un procédé industriel permettant d'éliminer ces inconvénients reste donc d'un intérêt primordial. Cependant, le choix d'un autre réactif approprié pour provoquer la déméthylation de l'éther méthylique de composés de structure Ia s'avère difficile.

On sait, en effet, que la famille des morphinanes alcaloides, étant donné sa structure complexe, nécessite une approche chimique particulière. La déméthylation en position 3 n'échappe pas à cette règle d'autant plus que les carbones 6, 7 et 14 peuvent être fonctionnalisés et/ou intervenir dans des liaisons carbone-carbone

susceptibles d'être doubles.

En conséquence, un réactif capable de provoquer cette déméthylation doit présenter une grande sélectivité entre les deux fonctions éther portées par les carbones 3 et 4 et laisser intactes les autres fonctions éventuellement présentes (cétones, alcènes, alcools...).

Pour ces raisons, chaque famille de dérivés morphiniques de structure Ia par exemple l'oxycodone, la codéine, la thébaïne et leurs analogues respectifs, possède pratiquement son réactif approprié, le tribomure de bore étant cependant celui qui apparaît comme le plus commun à ces différentes familles.

En conséquence, l'application d'une réaction déjà connue pour provoquer la déméthylation d'éther méthylique en général ne peut absolument pas être envisagée avec certitude au niveau des morphinanes de structure Ia.

A ce propos, on connaît la déméthylation de composés de formule relativement simple au moyen d'un couple acide sulfonique/dérivé de type sulfure, en l'occurence l'acide méthanesulfonique ou trifluoro méthanesulfonique et la méthionine. Cette technique est rapportée notamment dans J.C.S. Perkin I pp. 2288-2289 (1977). Les résultats obtenus en général avec cette méthode sont très divers (rendement de 30 à 90% en composés déméthylés) et peuvent être médiocres. C'est le cas de la p-méthoxyacétophénone : aucune réaction n'est enregistrée à 20°C et seulement 30% de p-hydroxyacétophénone sont obtenus après 72 heures à 60°C.

En conclusion, la généralisation de cette méthode au vu des résultats connus n'est absolument pas envisageable encore moins lorsqu'il s'agit de son application à des composés de structure complexe.

On a cependant trouvé que le procédé en question peut être utilisé valablement pour la déméthylation de certains dérivés de morphinane comportant la structure Ia, à savoir les composés de formule II ci-après, alors qu'il s'avère totalement inefficace pour la déméthylation d'autres familles de dérivés morphiniques de structure proche.

Ainsi, on a pu mettre en évidence, de manière surprenante, que la réaction de déméthylation en question peut être appliquée de manière efficace et sélective à la déméthylation de composés de structure Ia comportant un groupement hydroxyle en position 14 et un groupement hydroxyle ou cétonique en position 6 en vue d'obtenir les composés de formule I.

La présente invention, a en conséquence pour objet un procédé de préparation de composés de formule I qui consiste à faire réagir un équivalent d'un dérivé morphinique de formule générale :

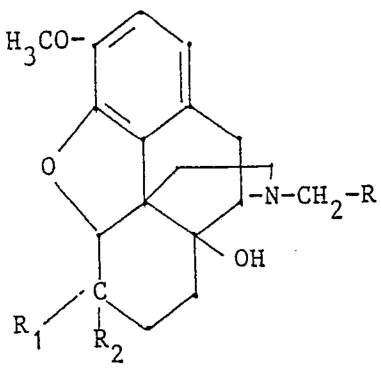

II

dans laquelle R, $R_1$ et $R_2$ ont la même signification que précédemment, avec 5 à 50 équivalents d'un acide sulfonique choisi parmi l'acide méthanesulfonique et l'acide trifluorométhanesulfonique en présence de 1 à 5 équivalents d'un sulfure de formule générale :

$W-S-W_1$ III

dans laquelle W et $W_1$, qui sont identiques ou différents, représentent chacun un radical alkyle linéaire en $C_1$-$C_4$, par exemple n-butyle, le groupement W étant éventuellement porteur d'un groupement amino et d'un groupement carboxylique, ou peuvent former avec l'atome de soufre un cycle saturé ayant jusqu'à 8 atomes de carbone, par exemple un groupe tétrahydrothiophényle.

Lorsque W est porteur d'un groupement amino et d'un groupement carboxylique, le composé de formule II est un acide aminé comportant une fonction sulfure. Un tel acide aminé est de préférence la méthionine.

Les dérivés morphiniques de formule II sont des composés connus ayant été décrits notamment dans les brevets US-A-4 141 897, 4 161 597 et 4 667 037 ou encore dans Chem. Abst. 104, 6055, 98, 204267 ou 75, 107912.

La réaction a lieu généralement en présence d'un large excès d'acide sulfonique, de préférence de 25 à 35 équivalents alors qu'un léger excès de sulfure de formule III est suffisant, c'est-à-dire de 1 à 2 équivalents.

Dans la plupart des cas, la température réactionnelle est de 20 à 60°C pouvant aller jusqu'à 80°C maximum : elle est comprise de préférence entre 30°C et 40°C.

En effet, à partir de 60°C, on commence à enregistrer, selon les cas, une dégradation du produit final déméthylé.

Sans être limitatives, les conditions opératoires suivantes seront généralement utilisées :

30 équivalents d'acide sulfonique

1,5 équivalent de sulfure de formule III

et ce, par équivalent de composé de formule II, la réaction ayant lieu de préférence entre 30 et 40° C pendant environ 15 heures.

Après purification du produit brut, ainsi obtenu, les eaux-mères de cristallisation contiennent, en quantité non négligeable du composé de formule I ainsi que du produit de formule II qu'il est possible de recycler.

D'autres acides protoniques forts ont été testés dans lecadre du procédé de l'invention mais se sont révélés totalement inefficaces pour provoquer la déméthylation.

Par exemple, on n'a pu enregistrer la formation de naloxone à partir de N-allylnoroxycodone (formule II dans laquelle R représente un radical vinyle, $CR_1R_2$ représente C = O) et de 15 équivalents d'acide chlorosulfonique ou sulfurique et de 1,5 équivalent de méthionine durant 1 à 8 h à 20° C. De même, la mise en oeuvre de résines superacides conduit également dans les mêmes conditions à un échec.

Le procédé de l'invention présente, en outre, certains avantages par rapport au procédé de déméthylation utilisant le tribromure de bore. Dans le cas par exemple de la formation de naloxone à partir de N-allylnoroxycodone, le procédé antérieur permet d'obtenir un rendement d'environ 62,5% en produit final. Cependant, le traitement du milieu réactionnel en vue de récupérer le produit final se révèle difficile à mettre en oeuvre étant donné la quantité importante de sels de bore formés au cours de la réaction.

Appliqué également à la déméthylation de la N-allylnoroxycodone, le procédé de l'invention fournit la naloxone également avec des rendements d'environ 63%. Cependant environ 10% supplémentaires de produit final peuvent être récupérés à partir du courant de recyclage. La récupération de la naloxone formée apparaît également plus facile en raison d'émulsions moins importantes au sein du milieu réactionnel. En outre, le procédé de l'invention se révèle plus rentable que le procédé antérieur étant donné le coût moindre des réactifs mis en oeuvre.

Comme indiqué précédemment, le procédé de l'invention peut être appliqué à la préparation des composés de formule I alors qu'il s'avère totalement inefficace pour l'obtention de dérivés morphiniques de structure proche.

A cet effet, on a pratiqué des essais de déméthylation de différents composés représentant différentes familles de dérivés morphiniques méthoxylés en utilisant les conditions de réaction préférées selon l'invention à savoir 30 équivalents d'acide méthanesulfonique et 1,5 équivalent de méthionine, la température et le temps de réaction étant indiqués ci-dessous :

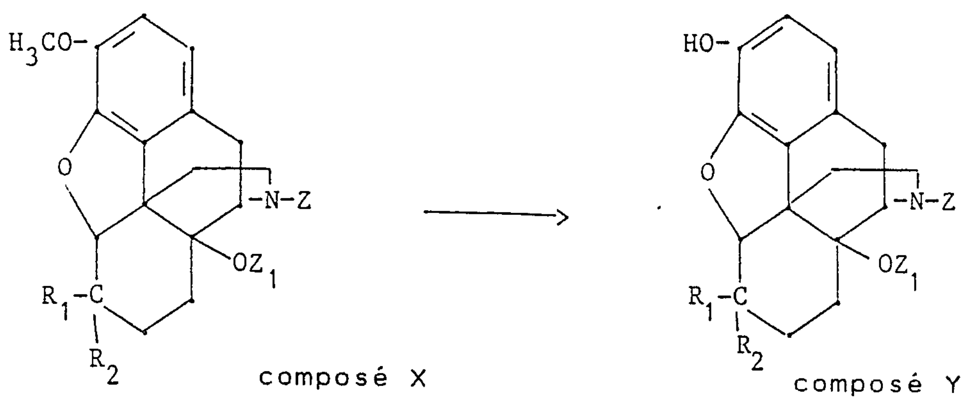

composé X

composé Y

a) Précurseurs de la naloxone

| Composé X | | | T(°C) | Temps (h) | Résultats |
|---|---|---|---|---|---|
| Z | $CR_1R_2$ | $Z_1$ | | | |
| $CH_3$ | C=O | $COCH_3$ | 20<br>40 | 12<br>7,5 | 59% du Composé Y où $R_1$=H *<br>(C.P.L. et C.C.M.) |
| $CO_2C_2H_5$ | C=O | $COCH_3$ | 20 | 20 | mélange complexe |
| H | C=O | H | 40 | 12 | 1% du Composé Y correspondant |
| $CO_2C_2H_5$ | C=O | H | 20 | 48 | mélange complexe contenant 18% du Composé Y et 6% du Composé X |

b) Précurseur de la naltrexone

| | | | | | |
|---|---|---|---|---|---|
| $CH_2$ 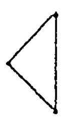 | $CH_2$ | H | 40 | 24 | mélange complexe contenant 15% du composé Y |
| | | | 20 | 6,5 | mélange complexe contenant 40% du composé Y |

* C.P.L. : chromatographie en phase liquide

C.C.M. : chromatographie sur couche mince

c) Précurseur de la buprenorphine

Le même procédé appliqué au composé ci-dessous :

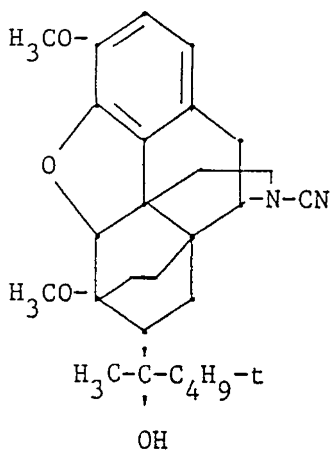

a montré qu'à 20° C, la réaction est très rapide et conduit à un mélange de produits. L'analyse par résonance magnétique nucléaire montre que la déméthylation n'a pas lieu mais que, par contre, le groupement tertiobutyle n'a pas résisté au milieu acide.

d) Précurseur de la morphine

Le procédé de l'invention appliqué à la codéine de formule :

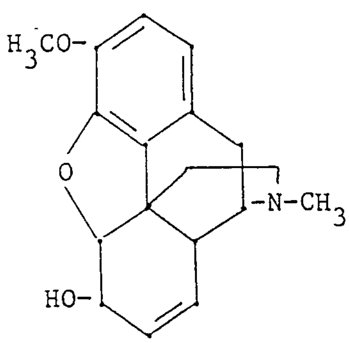

conduit à un mélange de nombreux produits parmi lesquels on peut noter par C.P.L. la présence de morphine (environ 10%), de produit de départ et de produit de réarrangement constitué par l'apocodéine.

e) Thébaine

On assite au même type de réaction avec la thébaine de formule :

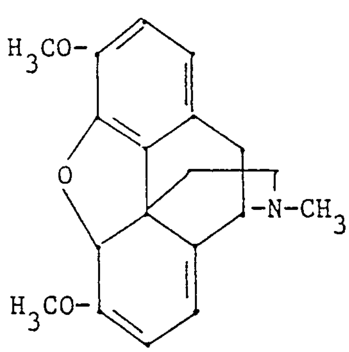

Dans un premier temps, le procédé de l'invention appliqué à 20° C pendant 3 heures fournit le produit réarrangé de formule :

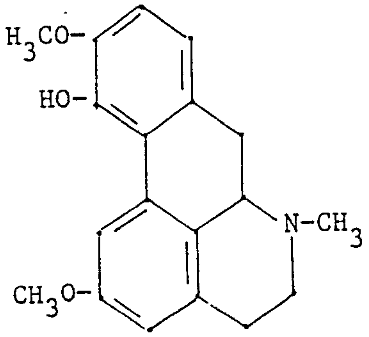

puis on observe une déméthylation partielle qui conduit après 3 jours à 20°C au dérivé de formule :

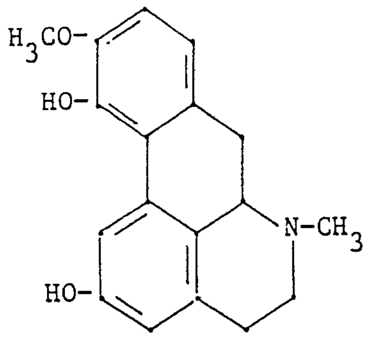

Ces résultats négatifs montrent clairement que l'application d'un procédé de déméthylation mettant en oeuvre l'acide méthanesulfonique ou trifluorométhanesulfonique et un sulfure de formule III, est tout à fait surprenante pour l'obtention des composés de formule I.

Les exemples, non limitatifs suivants, illustrent le procédé de l'invention :

## EXEMPLE 1

### Préparation du chlorhydrate d'époxy-4,5 dihydroxy-3,14 (propényl-2)-17(5 )morphinone-6 ou naloxone

a) Base libre

On ajoute 34,12 g (0, 1 mole) d'époxy-4,5 hydroxy-14 méthoxy-3 (propényl-2)-17(5 )morphinone-6 ou N-allylnoroxycodone à 288 g (3 moles) d'acide méthanesulfonique. La dissolution est exothermique. A la solution brun noir obtenue, on ajoute à 40° C en 15 minutes, 22,4 g (0, 15 mole) de DL-méthionine. On agite le milieu réactionnel à 40° C pendant 15,5 heures puis on alcalinise avec de l'ammoniaque jusqu'à pH = 8 à 9. On extrait à l'acétate d'éthyle pour obtenir 30,7 g d'un produit brut que l'on traite en solution chloroformique avec de l'alumine. Après recristallisation dans le toluène, on obtient 19,8 g de naloxone (P.F. = 177° C), ce qui représente un rendement de 60%.

On reclyle alors les eaux-mères de cristallisation qui renferment 9% de naloxone et 12% du produit de départ respectivement par rapport au produit de départ engagé.

b) Chlorhydrate

On dissout dans 30 ml d'acétone chaud, 5 g de base purifiée obtenue précédemment. Après concentration, à 10ml environ, on ajoute à chaud 5ml d'acide chlorhydrique 6N. On refroidit à -10° C, filtre, lave à l'acétone et sèche à 50° C sous vide.

On obtient ainsi 4,83 g de chlorhydrate de naloxone.

Rendement : 87%.

## EXEMPLE 2

### Préparation de la naloxone

On ajoute 34,12g (0,1 mole) de N-allylnoroxycodone à 288g (3 moles) d'acide méthanesulfonique. A la solution ainsi obtenue, on ajoute à 30° C en 15 minutes, 22,4g (0,15 mole) de DL-méthionine. On agite le milieu

réactionnel à 30°C pendant 15,5 h. puis on alcalinise avec de l'ammoniaque jusqu'à pH=8 à 9. On extrait à l'acétate d'éthyle et on traite avec de l'alumine une solution chloroformique du produit brut obtenu.

Après recristallisation dans le toluène on obtient la naloxone avec un rendement de 60% (P.F. : 176°C).

On recyle les eaux-mères toluéniques et on récupère de cette manière 7% de naloxone et 18% de produit de départ respectivement par rapport au produit de départ engagé.

## EXEMPLE 3

Préparation de la naloxone

On agite un mélange de 3,41g (0,01 mole) de N-allylnoroxycodone, 28,8g (0,3 mole) d'acide méthanesulfonique et 2,19g (0,015 mole) de sulfure de di-n- butyle durant 5 heures à 20°C, puis durant 14 heures à 40°C et enfin durant 8 heures à 60°C.

On alcalinise avec de l'ammoniaque jusqu'à pH=8 à 9 puis on extrait à l'acétate d'éthyle. On traite alors avec de l'alumine une solution chloroformique du produit ainsi obtenu puis on recristallise dans le toluène.

De cette manière, on obtient 1,64 g de naloxone, ce qui représente un rendement de 50%.

On recyle alors les eaux-mères de cristallisation qui renferment encore environ 9% de naloxone par rapport au produit de départ engagé.

## EXEMPLE 4

Préparation de la naloxone

On agite, pendant 20 heures à 40°C, un mélange de 3,41g (0,01 mole) de N-allylnoroxycodone, 28,8g (0,3 mole) d'acide méthanesulfonique et 1,32g (0,015 mole) de tétrahydrothiophène. On alcalinise avec de l'ammoniaque jusqu'à pH=8 à 9 puis on extrait à l'acétate d'éthyle. On traite alors avec de l'alumine une solution chloroformique du produit ainsi obtenu puis on recristallise dans le toluène.

On obtient de cette manière, 1,73g de naloxone, ce qui représente un rendement de 53%. On recycle alors les eaux-mères de cristallisation qui renferment encore 9 à 10% de naloxone par rapport au produit de départ engagé.

## EXEMPLE 5

Préparation de la naloxone

On ajoute 1,7g ($5.10^{-3}$ mole) de N-allylnoroxycodone à un mélange de 3,75g ($25.10^{-3}$ mole) d'acide trifluorométhanesulfonique, 1,12g ($7,5.10^{-3}$ mole) de DL-méthionine et 10ml d'acide trifluoroacétique.

On agite le milieu réactionnel à 20°C pendant 29 heures puis on alcalinise avec de l'ammoniaque jusqu'à pH=8 à 9. On extrait à l'acétate d'éthyle puis on traite, avec de l'alumine, une solution chloroformique du produit ainsi obtenu. Après recristallisation dans le toluène, on obtient 1,15g de naloxone, ce qui représente un rendement de 60%.

Les eaux-mères de cristallisation renferment encore 9% de naloxone par rapport au produit de départ engagé.

## EXEMPLE 6

Préparation de l'époxy-4,5 dihydroxy-3,14 méthyl-17(5 α)morphinone-6 ou oxymorphone

On porte à 40°C un mélange de 3,15g ($10^{-2}$ mole) d'époxy-4,5 hydroxy-14 méthoxy-3 méthyl-17(5α) morphinone-6 ou oxycodone, 28,3g ($3.10^{-1}$ mole) d'acide méthanesulfonique et 2,2g ($1,5.10^{-2}$ mole) de DL-méthionine. On agite pendant 12 heures à cette température puis on verse le milieu réactionnel sur de la glace. On alcalinise avec de l'ammoniaque jusqu'à pH = 8 à 9 puis on extrait au chlorure de méthylène. On lave les phases organiques à l'eau, on sèche sur sulfate de sodium et on amène à sec sous pression réduite. On purifie sur colonne de silice le produit brut (2,51g) ainsi obtenu en éluant au chloroforme pur puis avec un gradient de méthanol.

On obtient ainsi 2,17g d'oxymorphone ce qui représente un rendement de 72%. P.F. : 253°C.

Chromatographie sur couche mince : Rf = 0,60 (dichlorométhane/méthanol 7/3).

## EXEMPLE 7

Préparation de la cyclopropylméthyl-17 époxy-4,5 dihydroxy-3, 14(5α)morphinone-6 ou naltrexone

On forme un mélange de 1g ($2,8.10^{-3}$ mole) de cyclopropylméthyl-17 époxy-4,5 hydroxy-14 méthoxy-3 (5α)morphinone-6 ou méthylnaltrexone, 8,1g ($84.10^{-3}$ mole) d'acide méthanesulfonique et 0,63g ($4,2.10^{-3}$ mole) de DL-méthionine et on l'agite pendant 8 heures à 40°C puis pendant 15 heures à 20°C. On verse le milieu réactionnel dans un mélange d'ammoniaque et de glace jusqu'à obtenir un pH = 8 à 9. Après extraction à l'acétate d'éthyle, on lave les phases organiques à l'eau, on sèche sur sulfate de sodium et on amène à sec sous pression réduite.

On obtient ainsi 0,77g de naltrexone sous forme brute et on détermine le titre par chromatographie en phase liquide avec un échantillon authentique pour référence.

Titre obtenu : 82%

Rendement chimique de la déméthylation : 65,5%.

Chromatographie sur couche mince : Rf = 0,21 (toluène:acétone 7/3).

## EXEMPLE 8

Préparation du cyclobutylméthyl-17 époxy-4,5 trihydroxy-3,6,14 (5α, 6α) morphinane ou nalbuphine

On agite, pendant 8 heures à 40°C, un mélange de 1g ($2,71.10^{-3}$ mole) de cyclobutylméthyl-17 époxy-4,5 dihydroxy-6,14 méthoxy-3 (5α, 6α)morphinane ou méthylnalbuphine, 7,8g ($81.10^{-3}$ mole) d'acide méthanesulfonique et 0,61g ($4,1.10^{-3}$ mole) de DL-méthionine.

On verse le milieu réactionnel dans un mélange d'ammoniaque et de glace jusqu'à obtenir un pH = 8 à 9. Après extraction à l'acétate d'éthyle, on lave les phases organiques à l'eau, on sèche sur sulfate de sodium et on amène à sec sous pression réduite.

On obtient ainsi 0,97g de nalbuphine sous forme brute et on détermine le titre par chromatographie en phase liquide avec un échantillon authentique pour référence.

Titre obtenue : 47%

Rendement chimique de la déméthylation : 47%

Chromatographie sur couche mince : Rf = 0,50 (dichlorométhane/méthanol 85/15)

## EXEMPLE 9

Préparation de la naloxone

En utilisant le même procédé que celui décrit à l'Exemple 1 en partant de 0,1 mole de N-allylnoroxycodone mais en faisant varier les quantités d'acide méthanesulfonique et de méthionine et en modifiant éventuellement la température et la durée d'agitation du milieu réactionnel, on a obtenu les résultats suivants :

| Nombre de moles | | Température (°C) | Durée (h) | Rendement en naloxone (%) | Produit de départ non transformé (%) |
|---|---|---|---|---|---|
| Acide méthanesulfonique | Méthionine | | | | |
| 3 | 0,15 | 20 | 55 | 66 | 23 |
| 3 | 0,15 | 40 | 15 | 70 | 5,6 |
| 3 | 0,15 | 40 | 40 | 58 | 0 |
| 3 | 0,15 | 40<br>60 | 4<br>3 | 67 | 4,6 |
| 3 | 0,15 | 60 | 14 | 58 | 1 |
| 3 | 0,15 | 80 | 4 | 53 | 1 |
| 4 | 0,15 | 40 | 13 | 68 | 3 |
| 4 | 0,30 | 40 | 9 | 61 | 1,5 |
| 5 | 0,15 | 40 | 11 | 64 | 2 |

EXEMPLE 10

Préparation de la naloxone

On agite, pendant 21 heures à 20°C, un mélange de 1,7g ($5.10^{-3}$ mole)de N-allylnoroxycodone, 11,25g ($75.10^{-3}$ mole) d'acide trifluorométhanesulfonique, 1,35g ($75.10^{-3}$ mole) d'eau et 1,12g ($7,5.10^{-3}$ mole) de DL-méthionine.

On alcalinise avec de l'ammoniaque jusqu'à pH 8 à 9 puis on extrait à l'acétate d'éthyle. On traite alors, avec de l'alumine, le produit obtenu, mis en solution dans le chloroforme puis on recristallise dans le toluène.

On obtient ainsi 1,23g de naloxone ce qui représente un rendement de 64%. Les eaux-mères de cristallisation renferment encore 1% de naloxone par rapport au produit de départ engagé.

**Revendications**

1. Procédé de préparation de dérivés de morphinane de formule générale :

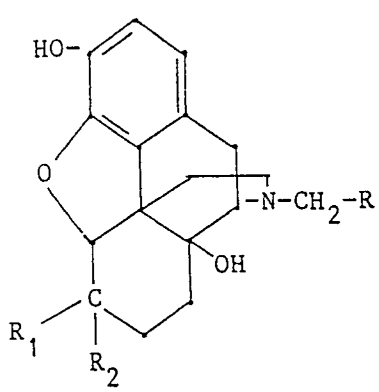

I

dans laquelle R représente l'hydrogène, un radical alkyle en $C_1$-$C_4$, cycloalkyle en $C_3$-$C_4$, alkényle en $C_2$-$C_4$ ou alkynyle en $C_2$-$C_4$, $R_1$ représente l'hydrogène, $R_2$ représente le radical hydroxyle ou $R_1$ et $R_2$, forment ensemble, avec l'atome de carbone auquel ils sont attachés, le groupe C=O, ainsi que de leurs sels pharmaceutiquement acceptables,

caractérisé en ce que l'on fait réagir un équivalent d'un dérivé morphinique de formule générale :

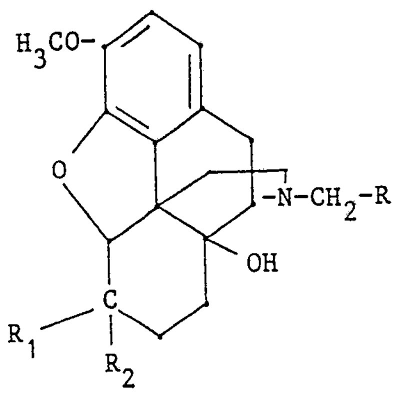

II

dans laquelle R, $R_1$ et $R_2$ ont la même signification que précédemment, avec 5 à 50 équivalents d'un acide sulfonique choisi parmi l'acide méthanesulfonique et l'acide trifluorométhanesulfonique en présence de 1 à 5 équivalents d'un sulfure de formule générale :

$W\text{-}S\text{-}W_1$ III

dans laquelle W et $W_1$, qui sont identiques ou différents, représentent chacun un radical alkyle linéaire en $C_1$-$C_4$, le groupement W étant éventuellement porteur d'un groupement amino et d'un groupement carboxylique, ou peuvent former avec l'atome de soufre un cycle saturé ayant jusqu'à 8 atomes de carbone.

2. Procédé selon la revendication 1, caractérisé en ce que l'on effectue la réaction à une température de 20 à 60° C.

3. Procédé selon l'une quelconque des revendications 1 et 2, caractérisé en ce que la température est comprise entre 30 et 40° C.

4. Procédé selon l'une quelconque des revendications 1 à 3, caractérisé en ce que l'on utilise de 25 à 35 équivalents d'acide sulfonique.

5. Procédé selon l'une quelconque des revendications 1 à 4, caractérisé en ce que l'on utilise de 1 à 2 équivalents de sulfure de formule III.

6. Procédé selon l'une quelconque des revendications 1 à 5, caractérisé en ce que le sulfure de formule III est le sulfure de di-n-butyle.

7. Procédé selon l'une quelconque des revendications 1 à 5, caractérisé en ce que le sulfure de formule III est le tétrahydrothiophène.

8. Procédé selon l'une quelconque des revendications 1 à 5, caractérisé en ce que le sulfure de formule III est la méthionine.

9. Procédé selon l'une quelconque des revendications 1 à 8, pour la préparation du composé de formule I dans laquelle R représente un radical vinyle et $CR_1R_2$ représente un groupe C=O.

10. Procédé selon l'une quelconque des revendications 1 à 8, pour la préparation du composé de formule I dans laquelle R représente l'hydrogène et $CR_1R_2$ représente un groupe C=O.

11. Procédé selon l'une quelconque des revendications 1 à 8, pour la préparation du composé de formule I dans laquelle R représente un radical cyclopropyle et $CR_1R_2$ représente un groupe C=O.

12. Procédé selon l'une quelconque des revendications 1 à 8 pour la préparation du composé de formule I dans laquelle R représene un radical cyclobutyle, $R_1$ représente l'hydrogène et $R_2$ représente un radical hydroxyle.

Office européen des brevets

# RAPPORT DE RECHERCHE EUROPEENNE

Numero de la demande

EP 89 40 2472

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | WO-A-8 000 841 (MALLINCKRODT INC.)<br>* Pages 1,2 * | 1 | C 07 D 489/08 |
| D,A | JOURNAL OF THE CHEMICAL SOCIETY, Perkin Transactions I, 1977, pages 2288-2289, Cambrige, GB; N. FUJII et al.: "Regioselective cleavage of aromatic methyl ethers by methanesulphonic acid in the presence of methionine"<br>* En entier * | 1 | |
| A | US-A-4 667 037 (E.I. DU PONT DE NEMOURS & CO.)<br>* Colonnes 1,2 * | 1 | |

DOMAINES TECHNIQUES RECHERCHES (Int. Cl.5)

C 07 D 489/00

Le présent rapport a été établi pour toutes les revendications

| Lieu de la recherche | Date d'achèvement de la recherche | Examinateur |
|---|---|---|
| LA HAYE | 29-11-1989 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)